# EUROPEAN PATENT APPLICATION

(11) **EP 3 238 675 A1**
(43) Date of publication of application: **01.11.2017**
(21) Application number: 16167609.3
(22) Date of filing: 29.04.2016
(51) Int. Cl.: A61F 13/532, A61F 13/15, B05C 1/08, B05C 19/04

(54) **APPARATUS FOR MAKING AN ABSORBENT STRUCTURE**

(71) Applicant: The Procter & Gamble Company, Cincinnati, OH 45202 (US)
(72) Inventor: BIANCHI, Ernesto Gabriel, 65 Schwalbach (DE); JACKELS, Hans Adolf, 53881 Euskirchen (DE); VAN DER KLUGT, Walter Pieter Hendrik Laurentius, 53881 Euskirchen (DE)
(74) Representative: Mather, Peter Geoffrey

(57) **Abstract**

The present invention relates to an apparatus for making an absorbent structure for an absorbent article, the apparatus comprising:
a printing roll comprising a plurality of cavities in the outer surface of the printing roll, each cavity having cavity sides which are formed by the walls of the cavity within the outer surface of the printing roll and a cavity base, and each cavity has a volume within the outer surface of the printing roll defined by the cavity sides and the cavity base, and wherein the plurality of cavities comprises cavities having different volume.

## Description

### FIELD OF THE INVENTION

The present invention relates to an apparatus for making an absorbent structure for an absorbent article, for example diapers, training pants, adult incontinence undergarments, feminine hygiene products, and the like.

### BACKGROUND OF THE INVENTION

Absorbent articles, such as disposable diapers, training pants, and adult incontinence undergarments, absorb and contain body exudates. They are also intended to prevent body exudates from soiling, wetting, or otherwise contaminating clothing or other articles, such as, bedding, that come in contact with the wearer. A disposable absorbent article, such as a disposal diaper, may be worn for several hours in a dry state or in a urine loaded state. Accordingly, efforts have been made toward improving the fit and comfort of the absorbent article to the wearer, both when the article is dry and when the article is fully or partially loaded with liquid exudates, while maintaining or enhancing the absorbing and containing functions of the article.

An important component of disposable absorbent articles is the absorbent core structure. Absorbent core structures typically include absorbent polymer material and cellulose fibers, or may absorbent polymer material free of cellulose fibers. The absorbent polymer material ensures that large amounts of bodily fluids, e.g. urine, can be absorbed by the absorbent article during its use and be locked away, thus providing low rewet and good skin dryness. The absorbent core structure is typically profiled, i.e. provided with regions of different capacity.

EP-A-1 621 165, EP-A-1 621 166 and EP-A-1 621 167, published on February 1st 2006, disclose a method of indirect printing of absorbent polymer (or gelling) material, AGM. This indirect printing is effected by means of a printing roll provided with cavities which are alternately filled with, and then discharge, absorbent polymer material. The cavities may be arranged in a repeating pattern which is designed to produce the desired absorbent core structure having the desired profile.

However certain constraints are encountered with the known printing roll apparatus. The present invention removes these constraints.

### SUMMARY OF THE INVENTION

The present invention relates to an apparatus for making an absorbent structure for an absorbent article, the apparatus comprising: a printing roll comprising a plurality of cavities in the outer surface of the printing roll, each cavity having cavity sides which are formed by the walls of the cavity within the outer surface of the printing roll and a cavity base, and each cavity has a volume within the outer surface of the printing roll defined by the cavity sides and the cavity base, and wherein the plurality of cavities comprises cavities having different volume.

Preferably each of cavities has the same depth, as defined by the distance from the outer surface to the cavity base, and wherein the plurality of cavities comprises cavities having different cross-sectional area.

### DETAILED DESCRIPTION OF THE INVENTION

The apparatus of the present invention is suitable for use in the manufacture of absorbent cores for absorbent articles such as disposable absorbent articles.

"Absorbent article" refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments, feminine hygiene products.

"Absorbent core" refers to a structure typically disposed between a topsheet and backsheet of an absorbent article for absorbing and containing liquid received by the absorbent article. The absorbent core may comprise one or more substrate layer, absorbent material disposed on the one or more substrate layer, and a thermoplastic adhesive composition on the absorbent material. The thermoplastic adhesive composition may be on the absorbent material and at least a portion of the one or more substrate layer. The absorbent core does not include an acquisition system, a topsheet, or a backsheet of the absorbent article. In a certain embodiment, the absorbent core would consist essentially of the one or more substrate layers, the absorbent material, the thermoplastic adhesive composition, and optionally the cover layer.

"Absorbent polymer material" or "absorbent gelling material" or "AGM" refers to cross linked polymeric materials that can absorb at least 10 times their weight of an aqueous 0.9% saline solution as measured using the Centrifuge Retention Capacity test (Edana 441.2-01).

"Absorbent polymer particles" is used herein to refer to an absorbent polymer material which is in particulate form so as to be flowable in the dry state.

A printing system may be used for making an absorbent core in accordance with the present invention. The system may comprise a printing unit for forming the absorbent structure. When the absorbent core is a laminate, the system may comprise a first printing unit for forming the first absorbent structure of the absorbent core and a second printing unit for forming the second absorbent structure of the absorbent core.

A printing unit may comprise a hopper for holding absorbent polymer material, a printing roll for transferring the absorbent polymer material to the substrate layer, a rotatable support roll for receiving the substrate layer, and thermoplastic adhesive material applicators for applying the thermoplastic adhesive material to the substrate and the absorbent polymer material thereon.

The printing roll comprises a rotatable drum and a plurality of absorbent polymer material reservoirs in an outer peripheral surface of the drum. The printing roll is designed to produce a profiled absorbent core. In particular, according to the present invention, the absorbent core is profiled in the transverse, i.e. cross-machine, direction. The absorbent core may also be profiled in the longitudinal, i.e. machine, direction. This effect is achieved by providing a plurality of cavities in the outer surface of the printing roll, each cavity having cavity sides which are formed by the walls of the cavity within the outer shell and a cavity base, and each cavity has a volume within the outer surface of the printing roll defined by the cavity sides and the cavity base. The plurality of cavities comprises cavities having different volume. Cavities having larger volume carry more absorbent polymer material and deliver more absorbent polymer material to the targeted zone and the cavities having smaller volume hold less particulate polymer material and deliver less absorbent polymer material to a target zone of the absorbent core. The distance between cavities may also vary across the transverse direction such that more densely clustered cavities deliver more absorbent material to the targeted zone and less densely clustered cavities deliver less absorbent polymer to the targeted zone.

In one embodiment of the present invention the printing roll comprises:
an outer shell comprising a plurality of apertures;
an inner shell which is permeable to air;
an apertured layer between the outer shell and the inner shell, the aperture layer having an outer surface adjacent to the outer shell and an inner surface adjacent to the inner shell;
and wherein the volume of each cavity is defined by the cavity sides and the cavity base formed by the outer surface of the aperture layer. This embodiment enables the application of a vacuum to the cavities by drawing vacuum through the aperture layer and the inner shell which supports the aperture layer. Other methods of delivering a varying profile of absorbent polymer basis weights to the absorbent core includes, but is not limited to, applying a higher vacuum in sections of the printing rolls where more absorbent polymer material is desired.

In operation, the printing system receives the substrate layer into the printing unit. A vacuum within the support roll draws the substrate layer against the vertical support grid and holds the substrate layer against the support roll. This presents an uneven surface on the substrate layer. Due to gravity, or by using the vacuum means, the substrate layer will follow the contours of the uneven surface and thereby the substrate layer will assume a mountain and valley shape. The absorbent particulate polymer material may accumulate in the valleys presented by the substrate layer. The support roll then carries the substrate layer past the rotating printing roll which transfers the absorbent polymer material from the hopper to the substrate layer in the grid pattern. A vacuum in the printing roll may hold the absorbent polymer material in the reservoirs until time to deliver the absorbent polymer material to the substrate layer. The vacuum may then be released or air flow through the air passages may be reversed to eject the absorbent polymer material from the reservoirs and onto the substrate layer. The absorbent polymer material may accumulate in the valleys presented by the substrate layer. The support roll then carries the printed substrate layer past the thermoplastic adhesive material applicators which apply thermoplastic adhesive material to cover the absorbent polymer material on the first substrate. This substantially immobilizes the absorbent polymer material in the desired profile.

In an exemplary embodiment of the present invention absorbent material is fed from a storage hopper, preferably by gravity, to the outer surface of the printing roll, or printing rolls. The outer surface comprises a pattern of reservoirs for holding absorbent material prior to depositing, or printing the absorbent material onto a substrate. Absorbent material is fed to the reservoir, the reservoir holds absorbent material in each of the cavities. The cavities may have a variety of shapes, including cylindrical, conical, or any other shape. In a preferred embodiment each of the cavities has a circular cross-section.

The reservoirs may contain one or more channels between the absorbent regions, the channel regions being substantially free of cavities. The channel regions preferably have a substantially constant width, defined by the width of the outer surface which is free of cavities, defined in the transverse, width-wise, direction of the outer shell.

As the printing roll is rotated the reservoir comprising absorbent material is transported to a depositing area at which the absorbent material is delivered from the cavities to a substrate, for example a nonwoven web, by a roll placed adjacent and in close proximity to the printing roll, for example substantially above the surface. The absorbent material may be deposited substantially continuously. The point or area where the absorbent material leaves the printing roll and transfers to the first moving endless surface is herein referred to as the depositing point or area. This point or area may contain a raised strip, e.g. each raised strip, mates with a mating strip, e.g. without direct contact.

In a particular embodiment of the present invention the apparatus comprises a first printing roll and a second printing roll. The first and second printing rolls may be arranged side by side so that two patterns of absorbent material are simultaneously printed onto two substrates. Both of the two substrates may then be combined to form a sandwich structure comprising absorbent material between the two substrates. The sandwich structure may be consolidated by applied pressure in a nip, e.g. between two rolls.

According to this embodiment of the invention the first printing unit may comprise an auxiliary adhesive applicator for applying an auxiliary adhesive to the substrate, a first rotatable support roll for receiving the first substrate, a first hopper for holding the absorbent particulate polymer material, a first printing roll for transferring the absorbent particulate polymer material from the hopper to the substrate, and a first thermoplastic adhesive material applicator for applying the fibrous thermoplastic adhesive material to the first substrate and the absorbent particulate polymer material land areas thereon.

The auxiliary adhesive applicator may be a nozzle system which can provide a relatively thin but wide curtain of thermoplastic adhesive material as suggested in WO2008/155699, but may instead advantageously comprise a slot coater for applying several slots of auxiliary adhesive simultaneously along the width of the substrate and fitted with a manifold to intermittently stop the delivery of the auxiliary adhesive so that there the auxiliary layer is not applied in the area of the substrate corresponding to the zones of lower absorbent material amount. The printing roll and adhesive applicator may be as further detailed in WO2008/155699. The absorbent structure obtained by the printing unit may be directly put in face to face relation with a second substrate, or may be combined with a second absorbent structure to form an absorbent core. This second absorbent structure may be formed on the second printing unit, which may be generally identical to the first printing unit. The second printing unit may comprise a second auxiliary adhesive applicator which may be a slot coater for applying an auxiliary adhesive to the substrate, a second rotatable support roll for receiving the substrate, a second hopper for holding absorbent particulate polymer material, a second printing roll for transferring the absorbent particulate polymer material to the substrate, and a thermoplastic adhesive material applicator for applying the thermoplastic adhesive material to the substrate and the absorbent particulate polymer land areas thereon.

The radius of the printing roll may depend on what absorbent structure is produced, e.g. what size, and for example how many structures are produced per cycle of the printing roll. For example, the printing roll may have a radius of at least 40 mm, or of at least 50 mm; it may be for example up to 300 mm, or up to 200 mm. In some embodiments, the radius of the printing roll is less than 50% of the radius of the first moving endless surface. In a preferred embodiment the radius of the printing roll is from 60mm to 80mm.

The printing roll may have any suitable width, but for example a width (for example in CD, hence perpendicular to MD) corresponding (substantially) to the width of the absorbent structure to be produced; this for example be at least 40 mm, or at least 60 mm, or for example up to 400 mm, or up to 200 mm.

The printing roll may have one or more reservoirs with a certain volume for receiving the absorbent material therein, and transporting it to and then depositing it on the supporting sheet on the first moving endless surface.

Each reservoir corresponds typically to an absorbent structure to be produced, as suitable for an absorbent article.

According to the present first and second supporting sheets are carried on first and second moving endless surfaces referred to herein as lay-down drums. Alternatively the moving endless surfaces could be belts.

The substrates are preferably sheets of nonwoven material. Absorbent material is deposited onto at least one, and preferably both, of the substrates from the printing roll, except in the regions corresponding to the raised strips and the mating strips.

The first printing roll preferably has one or more substantially longitudinally extending first mating strips, and the second printing roll preferably has corresponding longitudinally extending second mating strips. According to this embodiment of the invention pressure, P, is applied through the first and second mating strips to the first and second substrates at least within a part of the area of the channels, so as to adhere together the first and second substrates in the channel region.

In some embodiments, the first and second moving endless surfaces may for example have a speed of at least 1000, or at least 1200 parts per minute and/or a linear speed of at least 4.5 m/s, or at least 6 m/s, or at least 8 m/s, or at least 10 m/s.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. Apparatus for making an absorbent structure for an absorbent article, the apparatus comprising:
a printing roll comprising a plurality of cavities in the outer surface of the printing roll, each cavity having cavity sides which are formed by the walls of the cavity within the outer surface of the printing roll and a cavity base, and each cavity has a volume within the outer surface of the printing roll defined by the cavity sides and the cavity base,
**characterized in that** the plurality of cavities comprises cavities having different volume.

2. The apparatus according to Claim 1 wherein each of cavities has the same depth, as defined by the distance from the outer surface to the cavity base, and wherein the plurality of cavities comprises cavities having different cross-sectional area.

3. The apparatus according to claim 2 wherein each of the cavities has a circular cross-section.

4. The apparatus according any of the previous claims wherein cavities are unequally spaced with respect to the transverse, width-wise, direction of the outer shell so that the volume provided by the cavities varies across the width of the outer shell.

5. The apparatus according to any of the previous claims wherein the outer shell further comprises channel regions which are substantially free of cavities.

6. The apparatus according to claim 5 wherein the channel regions have a substantially constant width, defined by the width of the outer surface which is free of cavities, defined in the transverse, width-wise, direction of the outer shell.

7. The apparatus according to any of the previous claims wherein the printing roll comprises:
an outer shell comprising a plurality of apertures;
an inner shell which is permeable to air;
an apertured layer between the outer shell and the inner shell, the aperture layer having an outer surface adjacent to the outer shell and an inner surface adjacent to the inner shell and wherein the volume of each cavity is defined by the cavity sides and the cavity base formed by the outer surface of the aperture layer.
